(19) Europäisches Patentamt
European Patent Office
Office européen des brevets

(11) Veröffentlichungsnummer : **0 116 121**
**B1**

(12) # EUROPÄISCHE PATENTSCHRIFT

(45) Veröffentlichungstag der Patentschrift : **22.10.86**

(51) Int. Cl.⁴ : **C 08 F 2/50**, G 03 C 1/68

(21) Anmeldenummer : **83110564.8**

(22) Anmeldetag : **22.10.83**

(54) **Verfahren zur Photopolymerisation von Vinylverbindungen und photopolymerisierbares Material (II).**

(30) Priorität : **14.01.83 DE 3301011**

(43) Veröffentlichungstag der Anmeldung : **22.08.84 Patentblatt 84/34**

(45) Bekanntmachung des Hinweises auf die Patenterteilung : **22.10.86 Patentblatt 86/43**

(84) Benannte Vertragsstaaten : **AT CH DE FR GB LI**

(56) Entgegenhaltungen :
**FR-A- 1 545 208**
**FR-A- 2 273 042**
**GB-A- 1 529 863**

(73) Patentinhaber : **Kulzer & Co. GmbH**
**Philipp-Reis-Strasse 8**
**D-6393 Wehrheim (TS.)1 (DE)**

(72) Erfinder : **Schaefer, Roland Dr.**
**Merianweg 5**
**D-6382 Friedrichsdorf (DE)**

(74) Vertreter : **Heinen, Gerhard, Dr.**
**Heraeusstrasse 12-14**
**D-6450 Hanau/Main (DE)**

EP 0 116 121 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Verfahren zur Photopolymerisation von Vinylverbindungen in Gegenwart eines Photoinitiators aus

a) mindestens einem Photosensibilisator der allgemeinen Formel

$$A - \underset{\underset{O}{\|}}{C} - (X)_n - A$$

mit
$X = CO_2$, $C(R^1)$ $(R^2)$ oder $C(R^3)$ $(OR^4)$,
worin $R^1$, $R^2$, $R^3$ und $R^4$ H oder Kohlenwasserstoff-Reste bedeuten,
n = 0 oder 1,
A = gegebenenfalls substituierte Kohlenwasserstoff-Reste, die miteinander verbunden sein können und für n = 1 und $X = C(R^1)$ $(R^2)$ und für n = 0 aromatische Reste sind,
und

b) mindestens einem Reduktionsmittel und photopolymerisierbare Materialien.

Die Photopolymerisation findet vielseitige, auch technische Anwendung, zum Beispiel zur Härtung von Lacken und Überzügen, zur Herstellung von Druckplatten und beim Buchdruck.

Auch auf dem Dentalgebiet wird die Photopolymerisation angewandt. Photopolymerisierbare Materialien dienen zur Herstellung von Zahnfüllungen und -versiegelungen, von Kronen und Brücken und von künstlichen Gebissen (siehe zum Beispiel die britische Patentschrift 569 974 und die deutschen Offenlegungs- beziehungsweise Auslegeschriften 23 15 645, 23 57 324, 29 10 077 und 29 14 537).

In den britischen Patentschriften 1 408 265 und 1 529 863 werden photopolymerisierbare Materialien beschrieben, die als Photoinitiator ein Gemisch aus

a) mindestens einem Photosensibilisator der allgemeinen Formel

$$A - \underset{\underset{O}{\|}}{C} - (X)_n - A$$

mit
$X = CO$, $C(R^1)$ $(R^2)$ oder $C(R^3)$ $(OR^4)$, worin $R^1$, $R^2$, $R^3$ und $R^4$ H oder Kohlenwasserstoff-Reste bedeuten,
n = 0 oder 1,
A = gegebenenfalls substituierte Kohlenwasserstoff-Reste, die miteinander verbunden sein können und für n = 1 und $X = C(R^1)$ $(R^2)$ und für n = 0 aromatische Reste sind,
und
b) mindestens einem Reduktionsmittel der allgemeinen Formel

$$R - \overset{\overset{R}{|}}{M} - R,$$

worin M ein Element der Gruppe VB bedeutet, das gegebenenfalls mit zwei Resten R einen Ring bilden kann, und mindestens einer der Reste R in α-Stellung zu M eine CH-Gruppe aufweisen muß, wenn ein anderer eine aromatische Gruppe ist,
enthalten und durch Bestrahlung mit sichtbarem Licht oder durch UV-Strahlen ausgehärtet werden können.

Als Beispiele für die Photosensibilisatoren werden u. a. Biacetyl, Benzil, p,p'-Dialkoxybenzil, Benzoin und Campherchinon, für die Reduktionsmittel Propylamin, Dimethylaminoäthylmethacrylat, N,N'-Dimethylanilin und Piperidin genannt.

Die Verwendung von N,N'-disubstituierten cyclischen 1,3-Diazaverbindungen, besonders von N,N'-disubstituierten Imidazolidinen und Hexahydropyrimidinen als Beschleuniger in durch UV-Licht härtbarem Material wird in der europäischen Patentanmeldung 0 049 922 beschrieben.

Aus der deutschen Patentanmeldung DE-A-31 36 484 ist ein Verfahren zur Photopolymerisation von

Vinylverbindungen in Gegenwart von Ketonen und cyclischen beziehungsweise heterocyclischen Verbindungen, besonders von 5-substituierten Barbitursäuren, als Reduktionsmittel bekannt.

Es ist die Aufgabe der Erfindung, ein Verfahren zur Photopolymerisation von Vinylverbindungen in Gegenwart eines Photoinitiators aus einer Carbonylverbindung und einem Beschleuniger beziehungsweise Reduktionsmittel zu finden, das ein rasches Aushärten der Vinylverbindungen sowohl durch Bestrahlen mit UV-Licht als auch durch Bestrahlen mit sichtbarem Licht bewirkt. Die nach dem Verfahren hergestellten Polymerisate sollen eine gute Farbbeständigkeit besitzen.

Das die Lösung der Aufgabe darstellende Verfahren ist erfindungsgemäß dadurch gekennzeichnet, daß als Reduktionsmittel ein N-Alkyl- oder N-Aryl-pyrazol, -triazol oder -tetrazol verwendet wird.

Bewährt hat sich das erfindungsgemäße Verfahren, wenn als Reduktionsmittel ein 1-Arylpyrazolon-(5), ein 2-Aryl-2H-benzotriazol, ein 1-Alkyl-1H-tetrazol oder ein 1-Aryl-1H-tetrazol und als Photosensibilisator ein Diketon, vorzugsweise Campherchinon verwendet wird. Besonders bewährt hat sich die Verwendung eines Gemisches aus Camperchinon und einem Benzildialkylketal, vorzugsweise Benzildimethylketal, als Photosensibilisator.

Es war überraschend, daß die Geschwindigkeit der Photopolymerisation nach dem erfindungsgemäßen Verfahren der der in der britischen Patentschrift 1 408 265 beschriebenen Photopolymerisation entspricht. Die nach dem erfindungsgemäßen Verfahren erhaltenen Vinylpolymeren und -copolymeren besitzen jedoch den Vorteil der höheren Farbstabilität.

Das erfindungsgemäße Verfahren kann überall dort angewandt werden, wo monomere Vinylverbindungen beziehungsweise solche Verbindungen enthaltende Materialien durch Bestrahlen mit UV-Licht oder mit sichtbarem Licht polymerisiert werden sollen.

Unter Vinylverbindungen werden alle gebräuchlichen äthylenisch ungesättigten Verbindungen verstanden, besonders Acryl- und Methacrylsäureester ein- und mehrwertiger Alkohole, darunter auch die sogenannten Urethanacrylate und -methacrylate und das aus der US-PS 3 066 112 bekannte Bis-GMA, das Reaktionsprodukt aus Bis-Phenol A und Glycidylmethacrylat.

Den Vinylverbindungen beziehungsweise den diese Vinylverbindungen enthaltenden Materialien wird der Photosensibilisator zweckmäßigerweise in einer Menge von $10^{-2}$ bis 10 Gewichts-%, bezogen auf die Vinylverbindungen, zugesetzt; bevorzugt wird eine Menge von $10^{-1}$ bis 5 Gewichts-%. Das Reduktionsmittel kann in ebensolchen Mengen eingesetzt werden.

Besonders bewährt hat sich die Anwendung des erfindungsgemäßen Verfahrens auf dem Dentalgebiet für die Herstellung sowohl von Zahnfüllungen und -versiegelungen als auch von Kronen, Brücken und künstlichen Gebissen durch Polymerisation der Acrylsäureester und/oder Methacrylsäureester und gegebenenfalls anorganische Füllstoffe enthaltenden Materialien unter Bestrahlen mit UV-Licht oder mit sichtbarem Licht.

In den folgenden Beispielen werden Methacrylsäureester und anorganische Füllstoffe enthaltende photopolymerisierbare Materialien und deren Polymerisation gemäß der Erfindung beschrieben.

Die Schichtdicke der erhaltenen polymeren Formkörper wird gemessen; sie dient zur Beurteilung der Photoinitiator-Aktivität.

## Beispiele 1 bis 5

Eine Mischung aus
7,0 g Bis-GMA,
3,0 g Triäthylenglykoldimethacrylat,
30,0 g Lithiumaluminiumsilicat (85 Gewichts-% der Teilchen mit Teilchengröße < 15 µm),
1,0 g Aluminiumoxid und
X Photoinitiator (siehe Tabelle)

wird in eine Form (Innendurchmesser 6 mm, Höhe 10 mm) aus Delrin ®, ein Polyacetal-Kunststoff, gegeben, an der Oberfläche mit Polyesterfolie abgedeckt und 20 Sekunden lang mit dem Wolfram-Halogen-Lichtgerät Translux der Firma Kulzer in der Weise bestrahlt, daß das Lichtaustrittsfenster des Gerätes auf die Polyesterfolie gesetzt wird.

Dann wird der unpolymerisiert gebliebene Teil der Mischung entfernt und die Schichtdicke des polymerisierten Teils gemessen.

Art und Menge des Photoinitiators und die Schichtdicke werden in der Tabelle angegeben.
Benzildialkylketal = 2,2-Dialkoxy-1,2-diphenyläthanon
Benzildimethylketal = 2,2-Dimethoxy-1,2-diphenyläthanon

(Siehe Tabelle Seite 4 ff.)

**Patentansprüche**

1. Verfahren zur Photopolymerisation von Vinylverbindungen in Gegenwart eines Photoinitiators aus

a) mindestens einem Photosensibilisator der allgemeinen Formel

Tabelle

| Beispiel | Photoinitiator | Gewichts-% | Schichtdicke [mm] |
|---|---|---|---|
| 1 | Campherchinon<br>+<br>Benzildimethylketal<br>+<br>1-Phenyl-3-methyl-pyrazolon-(5) | 0,1<br>0,3<br>0,1 | 5,3 |
| 2 | Campherchinon<br>+<br>Benzildimethylketal<br>+<br>1-Phenyl-3,4-dimethyl-pyrazolon-(5) | 0,1<br>0,3<br>0,1 | 5,7 |
| 3 | Campherchinon<br>+<br>Benzildimethylketal<br>+<br>2-(2-Acetoxy-3,5-di-tert.-pentyl-phenyl)-2H-benzotriazol | 0,1<br>0,3<br>0,1 | 6,1 |
| | | | |

Tabelle (Fortsetzung)

| Beispiel | Photoinitiator | Gewichts-% | Schichtdicke [mm] |
|---|---|---|---|
| 4 | Campherchinon<br>+<br>Benzildimethylketal<br>+<br>1,5-Dimethyl-1H-tetrazol | 0,1<br><br>0,3<br><br>0,1 | 4,8 |
| 5 | Campherchinon<br>+<br>Benzildimethylketal<br>+<br>1-Phenyl-5-acetylthio-1H-tetrazol | 0,1<br><br>0,3<br><br>0,1 | 6,0 |
| | | | |

$$A - \underset{\underset{O}{\|}}{C} - (X)_n - A$$

mit

X = CO, $C(R^1)$ $(R^2)$ oder $C(R^3)$ $(OR^4)$, worin $R^1$, $R^2$, $R^3$ und $R^4$ H oder Kohlenwasserstoff-Reste bedeuten,

n = 0 oder 1,

A = gegebenenfalls substituierte Kohlenwasserstoff-Reste, die miteinander verbunden sein können und für n = 1 und X = $C(R^1)$ $(R^2)$ und für n = 0 aromatische Reste sind,

und

b) mindestens einem Reduktionsmittel,

dadurch gekennzeichnet, daß als Reduktionsmittel ein N-Alkyl- oder N-Aryl-pyrazol, -triazol oder -tetrazol verwendet wird.

2. Verfahren nach Anspruch 1, dadurch gekennzeichnet, daß als Reduktionsmittel ein 1-Arylpyrazolon-(5), ein 2-Aryl-2H-benzotriazol, ein 1-Alkyl-1H-tetrazol oder ein 1-Aryl-1H-tetrazol verwendet wird.

3. Verfahren nach Anspruch 1 oder 2, dadurch gekennzeichnet, daß als Photosensibilisator Campherchinon verwendet wird.

4. Verfahren nach Anspruch 3, dadurch gekennzeichnet, daß als Photosensibilisator zusätzlich ein Benzildialkylketal verwendet wird.

5. Photopolymerisierbares Material, das mindestens eine Vinylverbindung und einen Photoinitiator aus

a) mindestens einem Photosensibilisator der allgemeinen Formel

$$A - \underset{\underset{O}{\|}}{C} - (X)_n - A$$

mit

X = CO, $C(R^1)$ $(R^2)$ oder $C(R^3)$ $(OR^4)$, worin $R^1$, $R^2$, $R^3$ und $R^4$ H oder Kohlenwasserstoff-Reste bedeuten,

n = 0 oder 1,

A = gegebenenfalls substituierte Kohlenwasserstoff-Reste, die miteinander verbunden sein können und für n = 1 und X = $C(R^1)$ $(R^2)$ und für n = 0 aromatische Reste sind,

und

b) mindestens einem Reduktionsmittel

enthält, dadurch gekennzeichnet, daß das Reduktionsmittel ein N-Alkyl- oder N-Arylpyrazol, -triazol oder -tetrazol ist.

6. Photopolymerisierbares Material nach Anspruch 5, dadurch gekennzeichnet, daß das Reduktionsmittel ein 1-Arylpyrazolon-(5), ein 2-Aryl-2H-benzotriazol, ein 1-Alkyl-1H-tetrazol oder ein 1-Aryl-1H-tetrazol ist.

7. Photopolymerisierbares Material nach Anspruch 6, dadurch gekennzeichnet, daß der Photosensibilisator Campherchinon ist.

8. Photopolymerisierbares Material nach Anspruch 7, dadurch gekennzeichnet, daß der Photosensibilisator zusätzlich ein Benzildialkylketal enthält.

9. Photopolymerisierbares Material nach Anspruch 8, dadurch gekennzeichnet, daß es als Vinylverbindung Acrylsäureester und/oder Methacrylsäureester und als Reduktionsmittel ein 1-Aryl-pyrazolon-(5) enthält.

10. Photopolymerisierbares Material nach Anspruch 8, dadurch gekennzeichnet, daß es als Vinylverbindung Acrylsäureester und/oder Methacrylsäureester und als Reduktionsmittel ein 2-Aryl-2H-benzotriazol enthält.

## Claims

1. Process for the photopolymerisation of vinyl compounds in the presence of a photo-initiator consisting of :

a) at least one photosensitiser having the general formula

$$A - \underset{\underset{O}{\|}}{C} - (X)_n - A$$

with

$x = CO$, $C(R^1)(R^2)$ or $C(R^3)(OR^4)$, in which $R^1$, $R^2$, $R^3$ and $R^4$ denote H or hydrocarbon radicals,

$n = 0$ or 1,

A = possibly substituted hydrocarbon radicals which may be combined with each other, and which for $n = 1$ and $X = C(R^1)(R^2)$ and for $n = 0$ are aromatic radicals,

and

b) at least one reducing agent,

characterised in that an N-alkyl or N-aryl-pyrazole, -triazole or -tetrazole, is utilised as a reducing agent.

2. Process according to claim 1, characterised in that a 1-arylpyrazolone-(5), a 2-aryl-2H-benzotriazole, a 1-alkyl-1H-tetrazole or a 1-aryl-1H-tetrazole is utilised as a reducing agent.

3. Process according to claim 1 or 2, characterised in that camphor quinone is utilised as a photosensitiser.

4. Process according to claim 3, characterised in that a benzyl dialkyl ketal is complementarily utilised as a photosensitiser.

5. Photopolymerisable material which contains a vinyl compound and a photoinitiator consisting of

a) at least one photosensitiser having the general formula

$$A - \underset{\underset{O}{\|}}{C} - (X)_n - A$$

with

$x = CO$, $C(R^1)(R^2)$ or $C(R^3)(OR^4)$, in which $R^1$, $R^2$, $R^3$ and $R^4$ denote H or hydrocarbon residues,

$n = 0$ or 1,

A = possibly substituted hydrocarbon residues which may be combined and which, for $n = 1$ and $x = C(R^1)(R^2)$ and for $n = 0$, are aromatic radicals,

and

b) at least one reducing agent,

characterised in that the reducing agent is an N-alkyl- or N-arylpyrazole, -triazole or -tetrazole.

6. Photopolymerisable material according to claim 5, characterised in that the reducing agent is a 1-arylpyrazolone-(5), a 2-aryl-2H-benzotriazole, a 1-alkyl-1H-tetrazole or a 1-aryl-1H-tetrazole.

7. Photopolymerisable material according to claim 6, characterised in that the photosensitiser is camphor quinone.

8. Photopolymerisable material according to claim 7, characterised in that the photosensitiser complementarily contains a benzyl dialkylketal.

9. Photopolymerisable material according to claim 8, characterised in that it contains acrylic acid ester and/or methacrylic acid ester as a vinyl compound, and a 1-aryl-pyrazolone-(5) as a reducing agent.

10. Photopolymerisable material according to claim 8, characterised in that it contains acrylic acid ester and/or methacrylic acid ester as a vinyl compound, and a 2-aryl-2H-benzotriazole as a reducing agent.

**Revendications**

1. Procédé pour la photopolymérisation de composés vinyliques en présence d'un photo-inducteur consistant en :

a) au moins un photosensibilisateur de formule générale

$$A - \underset{\underset{O}{\|}}{C} - (X)_n - A$$

dans laquelle

$X = CO$. $C(R^1)(R^2)$ ou $C(R^3)(OR^4)$, où $R^1$, $R^2$, $R^3$ et $R^4$ représentent H ou des restes d'hydrocarbures,

n = 0 ou 1,
A = des restes d'hydrocarbures éventuellement substitués qui peuvent être reliés entre eux et, pour n = 1 et X = C($R^1$) ($R^2$) et pour n = 0, des restes aromatiques,
et
b) au moins un agent réducteur,
caractérisé en ce que l'on utilise comme agent réducteur un N-alkyl- ou N-arylpyrazole, -triazole ou -tétrazole.

2. Procédé selon la revendication 1, caractérisé en ce que l'on utilise comme agent inducteur une 1-arylpyrazolone-(5), un 2-aryl-2H-benzotriazole, un 1-alkyl-1H-tétrazole ou un 1-aryl-1H-tétrazole.

3. Procédé selon la revendication 1 ou 2, caractérisé en ce que l'on utilise comme photosensibilisateur la camphoquinone.

4. Procédé selon la revendication 3, caractérisé en ce que l'on utilise en outre comme photosensibilisateur un dialkylacétal de benzile.

5. Matériau photopolymérisable, qui contient au moins un composé vinylique et un photo-inducteur consistant en :
a) au moins un photosensibilisateur de formule générale

$$A - \underset{\underset{O}{\|}}{C} - (X)_n - A$$

dans laquelle
X = CO, C($R^1$) ($R^2$) ou C($R^3$) ($OR^4$) où $R^1$, $R^2$, $R^3$ et $R^4$ représentent H ou des restes d'hydrocarbures,
n = 0 ou 1,
A = des restes d'hydrocarbures éventuellement substitués, qui peuvent être reliés entre eux et, pour n = 1 et X = C($R^1$) ($R^2$) et pour n = 0, des restes aromatiques,
et
b) au moins un agent réducteur,
caractérisé en ce que l'agent réducteur est un N-alkyl- ou N-arylpyrazole, -triazole ou -tétrazole.

6. Matériau photopolymérisable selon la revendication 5, caractérisé en ce que l'agent réducteur est une 1-arylpyrazolone-(5), un 2-aryl-2H-benzotriazole, un 1-alkyl-1H-tétrazole ou un 1-aryl-1H-tétrazole.

7. Matériau photopolymérisable selon la revendication 6, caractérisé en ce que le photosensibilisateur est la camphoquinone.

8. Matériau photopolymérisable selon la revendication 7, caractérisé en ce que le photosensibilisateur contient outre un dialkylacétal du benzile.

9. Matériau photopolymérisable selon la revendication 8, caractérisé en ce qu'il contient comme composé vinylique un ester d'acide acrylique et/ou un ester d'acide méthacrylique et comme agent réducteur une 1-aryl-pyrazolone-(5).

10. Matériau photopolymérisable selon la revendication 8, caractérisé en ce qu'il contient comme composé vinylique un ester d'acide acrylique et/ou un ester d'acide méthacrylique et comme agent réducteur un 2-aryl-2H-benzotriazole.